Europäisches Patentamt

European Patent Office

Office européen des brevets

(19)

(11) Veröffentlichungsnummer: **0 009 132**
**A1**

(12) # EUROPÄISCHE PATENTANMELDUNG

(21) Anmeldenummer: **79103118.0**

(22) Anmeldetag: **24.08.79**

(51) Int. Cl.³: **A 61 F 13/20,** D 04 H 1/02,
**D 04 H 1/60**

(30) Priorität: **05.09.78 DE 2838618**

(43) Veröffentlichungstag der Anmeldung: **02.04.80**
**Patentblatt 80/7**

(84) Benannte Vertragsstaaten: **AT CH DE FR GB IT SE**

(71) Anmelder: **BAYER Aktiengesellschaft, Zentralbereich
Patente,Marken und Lizenzen Bayerwerk, D-5090
Leverkusen 1 (DE)**

(72) Erfinder: **Graf, Heinz, Dipl.-Ing., Eulenweg 2, D-4047
Dormagen (DE)**
Erfinder: **Dorsch, Peter, Dr., Bahnhofstrasse 39,
D-4047 Dormagen 1 (DE)**

(54) Verfahren zur Verfestigung von Wattebändern aus Synthesefasern für die Tampon-Herstellung.

(57) Die Erfindung betrifft ein Verfahren zur Herstellung von verfestigten Wattebändern zur Tampon-Herstellung aus Synthesefasern, wobei den Synthesefasern Stoffe zugemischt werden, im allgemeinen in Mengen von 1 bis 50 Gew.-%, bezogen auf die Mischung, die unter Temperatureinwirkung klebefähig werden und daß im Zuge der Bandherstellung eine Verklebung herbeigeführt wird.

EP 0 009 132 A1

BAYER AKTIENGESELLSCHAFT    5090 Leverkusen, Bayerwerk

Zentralbereich                Dn/bc/Kü
Patente, Marken und Lizenzen


Verfahren zur Verfestigung von Wattebändern aus Synthesefasern für die Tampon-Herstellung

---

Bei der Herstellung von Tampons für die Frauen-Hygiene
werden Wattebänder auf der Tampon-Maschine verarbeitet.
Um die übliche Lagerhaltung diese Wattebänder und ihre
störungsfreie Verarbeitung auf der Tampon-Maschine zu
gewährleisten, müssen diese Wattebänder zuvor verfestigt
werden.

Die auf diesem Einsatzgebiet üblicherweise verwendeten
Wattebänder aus Baumwollfasern oder Regenerat-Zellulosefasern lassen sich auf einfache Weise durch eine Kalanderpressung verfestigen. Dadurch, daß diese Fasern im
Vergleich zu Synthesefasern eine geringere Kräuselung,
geringere Biegesteifigkeit, geringere Bauschelastizität
und eine andersartige Oberflächenbeschaffenheit aufweisen, gelingt diese Verfestigung durch Kalanderpressung

- 2 -

leicht und formbeständig.

Setzt man jedoch unter Beibehaltung des in dieser Technik
üblichen Produktionsschemas: Krempelvliesherstellung -
Vliesverdichtung zum Watteband - Kalanderpressung - Bandablage (was wegen des bei den Herstellern vorhandenen
Maschinenparks wünschenswert wäre) statt der zellulosischen Fasern Synthesefasern ein, so lassen sich durch
einfaches mechanisches Verpressen keine zufriedenstellenden Bandfestigkeiten erzielen, da die Bänder wieder
aufspringen. Daher ist ein rationelles Weiterverarbeiten auf der Tampon-Maschine nicht möglich.

Es wurde nun gefunden, daß beim Einsatz von Synthesefasern ausreichende Bandfestigkeiten dadurch erzielt
werden können, daß man den Synthesefasern durch Temperatureinwirkung klebefähig werdende Stoffe beimischt
und im Zuge der Herstellung des Wattebandes eine Verklebung herbeiführt.

Die Erfindung betrifft daher ein Verfahren zur Herstellung von verfestigten Wattebändern zur Tampon-
Herstellung aus Synthesefasern das dadurch gekennzeichnet ist, daß den Synthesefasern Stoffe zugemischt werden die unter Temperatureinwirkung klebefähig werden
und daß im Zuge der Bandherstellung eine Verklebung
herbeigeführt wird.

Als zur Verklebung geeignete Stoffe kommen solche Stoffe
in Betracht, die bei Temperaturen klebefähig werden, die

Le A 19 104

- 3 -

nicht irgendeinen negativen Effekt für die Wattebandherstellung mit sich bringen.

Für das erfindungsgemäße Verfahren sind vorzugsweise folgende Stoffe geeignet:

Teilverseifte Äthylen-Vinylacetat-Copolymere (25 bis 75 %
verseift; Fp. 140 bis 180°C);
teilvernetzte Polyvinylalkohole (Fp. bis 140°C);
Polyvinylchlorid und nachchloriertes Polyvinylchlorid
(Fp. 80 bis 140°C);
Vinylchlorid-Vinylacetat-Copolymere (Fp. 80 bis 160°C);
Copolyamide (Fp. 110 bis 145°C);
Copolyester auf Basis kurzkettiger aliphatischer Dicarbonsäuren und Diole (Fp. 50 bis 60°C) und
Polykondensate auf Basis aliphatischer Diglykole und Dicarbonsäuren (Fp. 40 bis 60°C).

Diese Stoffe können den Synthesefasern in Form von Fasern
(Schmelzklebefasern) oder auch in Form von Pulvern zugemischt werden.

Im Hinblick auf das Einsatzgebiet sollte bei der Auswahl
der Stoffe auf physiologische Unbedenklichkeit geachtet
werden.

Diese Stoffe werden den Synthesefasern im allgemeinen in
Mengen von 1 bis 50 Gew.-%, bezogen auf die Mischung,
zugemischt. Vorzugsweise beträgt die Menge dieser Schmelz-

Le A 19 104

- 4 -

klebestoffe 2 bis 35 Gew.-%.

Die Verklebung selbst kann in einfacher und an sich bekannter Weise durch den Einsatz von Heizzonen (beispielsweise IR-Felder oder Heizplatten) oder eines beheizbaren
Preßkalanders durchgeführt werden.

Als Synthesefasern die im Rahmen der vorliegenden Erfindung Verwendung finden, kommen vorzugsweise solche in
Betracht, die eine im Hinblick auf das Einsatzgebiet
ausreichende Flüssigkeitsspeicherung und Saugfähigkeit
aufweisen. Unter diesen sind besonders die in der DE-OS
2 554 124 beschriebenen Fasern, insbesondere die auf Acrylnitrilbasis, besonders bevorzugt.

Le A 19 104

- 5 -

Patentansprüche

1) Verfahren zur Herstellung von verfestigten Wattebändern zur Tamponherstellung aus Synthesefasern, dadurch gekennzeichnet, daß den Synthesefasern Stoffe zugemischt werden, die unter Temperatureinwirkung klebefähig werden und daß im Zuge der Bandherstellung eine Verklebung herbeigeführt wird.

2) Verfahren nach Anspruch 1, dadurch gekennzeichnet, daß den Synthesefasern 1 bis 50 Gew.-%, bezogen auf die Mischung, klebefähiger Stoffe zugemischt werden.

3) Verfahren nach den Ansprüchen 1 und 2, dadurch gekennzeichnet, daß die Synthesefasern eine ausreichende Flüssigkeitsspeicherung und Saugfähigkeit aufweisen.

Le A 19 104

| | Europäisches Patentamt | **EUROPÄISCHER RECHERCHENBERICHT** | 0009132 Nummer der Anmeldung |
|---|---|---|---|

EP 79 103 118.0

| **EINSCHLÄGIGE DOKUMENTE** | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| | DE − A1 − 2 742 208 (ROHM AND HAAS CO.) <br> * Patentansprüche 16 und 3 * <br><br> −− | 1,3 | A 61 F 13/20 <br> D 04 H 1/02 <br> D 04 H 1/60 |
| A | DE − A1 − 2 736 816 (COLGATE-PALMOLIVE CO.) <br> * Patentansprüche 16 und 18; Seite 18, Zeilen 7 bis 10 * <br><br> −− | 1 | |
| A | DE − A1 − 2 520 899 (MO OCH DOMSJÖ AB) <br> * Seite 5, 2. Absatz bis Seite 7 * <br><br> −− | 1 | RECHERCHIERTE SACHGEBIETE (Int. Cl.3) |
| A | DE − A − 1 922 089 (ARTOS DR.−ING. MEIER-WINDHORST KG) <br> * Seite 1, Zeilen 8 bis 11; Seite 2, Zeilen 1 bis 4 * <br><br> −− | 1 | A 61 F 13/00 <br> D 04 H 1/00 |
| A | DE − A − 1 560 872 (KALLE AG) <br> * Anspruch 8 * <br><br> −− | 1 | |
| A | DE − B − 1 269 990 (GUSTIN-BACON MANU-FACTURING CO.) <br> * Patentanspruch * <br><br> −− | 1 | KATEGORIE DER GENANNTEN DOKUMENTE |
| A | DE − B − 1 264 685 (MO OCH DOMSJÖ AB) <br> * Spalte 1, Zeilen 11 bis 20 * <br><br> −− | 1 | X: von besonderer Bedeutung <br> A: technologischer Hintergrund <br> O: nichtschriftliche Offenbarung <br> P: Zwischenliteratur <br> T: der Erfindung zugrunde liegende Theorien oder Grundsätze |
| A | DE − C − 1 255 925 (VEREINIGTE PAPIER-WERKE SCHICKEDANZ & CO.) <br> * Patentanspruch * <br><br> −− <br> ./.. | 1 | E: kollidierende Anmeldung <br> D: in der Anmeldung angeführtes Dokument <br> L: aus andern Gründen angeführtes Dokument |
| X | Der vorliegende Recherchenbericht wurde für alle Patentansprüche erstellt. | | &: Mitglied der gleichen Patent-familie, übereinstimmendes Dokument |
| Recherchenort <br> Berlin | Abschlußdatum der Recherche <br> 17-12-1979 | Prüfer <br> ARENDT | |

EPA form 1503.1   06.78

Europäisches
Patentamt

**EUROPÄISCHER RECHERCHENBERICHT**

00091.32

EP 79 103 118.0
- Seite 2 -

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | US - A - 3 716 430 (I. CROON et al.) <br><br> * ganzes Dokument * <br><br> --- | 1 |
| A | US - A - 3 200 181 (B. RUDLOFF) <br><br> * ganzes Dokument * <br><br> --- | 1 |
| A | MELLIAND TEXTILBERICHTE, Band 53, Heft Nr. 6, 1972, Heidelberg <br> H. GILCH et al. "Bikomponent-Polyamid-fasern" <br> Seiten 631 bis 636 <br> * Bild 1, verfestigte Vliese; Seite 632, 2. Spalte * <br><br> -- | 1 |
| A | MELLIAND TEXTILBERICHTE, Band 53, Heft Nr. 6, 1972, Heidelberg <br> W. von LANGENTHAL et al. "Dispersions-binder und Faser - ein Beitrag zur Vliesverfestigung" <br> Seiten 687 bis 692 <br> * ganzes Dokument * <br><br> ---- | 1 |

**KLASSIFIKATION DER ANMELDUNG (Int.Cl.3)**

**RECHERCHIERTE SACHGEBIETE (Int. Cl.3)**